# EUROPÄISCHE PATENTSCHRIFT

(11) **EP 0 762 859 B1**
(45) Veröffentlichungstag und Bekanntmachung des Hinweises auf die Patenterteilung: **07.01.1998**
(21) Anmeldenummer: 95934608.1
(22) Anmeldetag: 17.10.1995
(51) Int. Cl.: A61F 7/00

(54) **WÄRMEDECKE ZUR VERWENDUNG BEI ÄRZTLICHEN OPERATIONEN**
HEATING BLANKET FOR USE DURING MEDICAL OPERATIONS
COUVERTURE CHAUFFANTE S'UTILISANT LORS D'INTERVENTIONS MEDICALES

(30) Priorität: 19.10.1994 DE 9416787 U; 12.10.1995 DE 29516246 U
(43) Veröffentlichungstag der Anmeldung: 19.03.1997
(73) Patentinhaber: John, Christine, 48231 Warendorf (DE)
(72) Erfinder: John, Christine, 48231 Warendorf (DE)
(74) Vertreter: Schirmer, Siegfried, Dipl.-Ing.
(86) Internationale Anmeldenummer: DE9501434
(87) Internationale Veröffentlichungsnummer: WO9612454

(56) Entgegenhaltungen:
- WO-A-94/03131
- WO-A-95/20371
- DE-U- 9 319 969
- DE-U- 9 421 053
- Kunststoff-Lexikon , Carl Hanser Verlag - München , S.54

## Beschreibung

Die Erfindung betrifft eine Wärmedecke zur Verwendung bei ärztlichen Operationen, die auf einen Teil eines liegenden Patienten aufzulegen und die mit temperierter Luft beschickbar ist. Die Wärmedecke besteht aus zwei randseitig zu einem Hohlraum verbundenen Zuschnitten aus flexiblem Material, von denen der oben liegende Zuschnitt aus einer luftdicht mit Kunststoff beschichteten textilen Bahn und der untere, dem Patienten zugewandte Zuschnitt aus einem dichten, jedoch noch luftdurchlässigen textilen Gewebe besteht, bei dem dann, wenn sich in dem Hohlraum Luft unter leichtem Überdruck befindet, diese aus der Unterseite der Wärmedecke, d. h. dem unteren Zuschnitt, flächenhaft verteilt austritt, und die mit wenigstens einem, vorzugsweise zwei in Abstand angeordneten, wechselseitig verschließbaren Lufteintrittsstutzen zum Anschluß an ein Luftgebläse versehen ist.

Es ist bekannt, derartige Wärmedecken anstelle einer zur Einmalverwendung aus einer Zellstoff-Vliesbahn und einer aufgelegten, luftundurchlässig gemachten Papierbahn herzustellen (Gebrauchsmuster 93 19 969).

Die bei einer Operation auftretenden Körperflüssigkeiten sowie Desinfektionsflüssigkeiten ergeben oftmals Situationen, bei denen die Wärmedecke mehr oder weniger stark verschmutzt wird, so daß ihre Verwendung bei einem weiteren Patienten nicht angebracht ist. Die auf dem Markt befindlichen Wärmedecken für die Einmalverwendung sind aber sehr kostspielig, da sie aus einer relativ großen Menge Material hergestellt sind, jeweils über größere Strecken geliefert werden müssen und eine relativ kostspielige Verarbeitung erforderlich machen. Die Kosten erhöhen sich noch dadurch, daß die Wärmedecken über spezielle Krankenhaus-Entsorgungsunternehmen entsorgt werden müssen.

Bei der bekannten, mehrfach verwendbaren Wärmedecke wird an deren Oberseite ein desinfizierend waschbares, luftdichtes Gewebe verwendet. Verwendet wird ein polyurethan- und/oder silikonbeschichtetes Polyamidgewebe ("Spinnakertuch"). Die bekannte Decke ist dafür ausgerüstet, im desinfizierenden Waschverfahren, d. h. im sogenannten Desinfektions-Waschverfahren, behandelt zu werden. Darunter wird laut "Richtlinie für Krankenhaushygiene und Infektionsprävention", herausgegeben vom Robert-Koch-Institut, Gustav Fischer Verlag, Stuttgart/Jena, gemäß Kapitel 7.2/3.6.4, ein Waschverfahren verstanden, bei dem in einer Flotte Desinfektions- und Waschmittel gemischt werden. Beim thermischen Desinfektions-Waschverfahren werden Flotte und Wäsche durch die Einwirkung einer Temperatur von 85° bis 90° C desinfiziert. Die Zusätze zur Flotte dienen dazu, die Verunreinigungen aufzuschließen, vom Textil abzulösen und während der Erhitzung in Suspension zu halten. Bei diesem Verfahren erfolgt die Desinfektion durch die Einwirkung eines chemischen Desinfektionsmittels auf die Wäsche. Die Flotte und die Wäsche werden erwärmt, um die Wirksamkeit des chemischen Mittels zu verstärken und zu beschleunigen.

Nachteilig bei dem Desinfektion-Waschverfahren ist, daß hohe Mengen an desinfektionsmittelhaltigen Wässern anfallen, die bei dem Waschpersonal allergische oder toxische Reaktionen verursachen können, die sehr schwer in einem Klärwerk zu verarbeiten sind und die relativ hohe Kosten durch die Zuführung von Desinfektionsmitteln erfordern.

Im Krankenhausbetrieb wird daher das desinfektionsmittelfreie Kochen der Wäsche oder die Dampfsterilisation bevorzugt. Für diese Wasch- und Sterilisierverfahren, die ohne Desinfektionsmittel arbeiten, ist die bekannte wiederverwendbare Warmluftdecke nicht geeignet. Überdies ist das zu verwendende "Spinnakertuch" relativ schwer und sperrig.

Es stellt sich demnach die Aufgabe, eine Wärmedecke anzugeben, die sich besser, in den Krankenhausbetrieb einfügen läßt, die ohne Einsatz von Desinfektionsmitteln desinfizierbar ist und die vorzugsweise so leicht und anschmiegsam gemacht werden kann, daß sie weder den Patienten noch das Operationsteam behindert.

Diese Aufgabe wird von einer Wärmedecke der eingangs genannten Art erfindungsgemäß dadurch gelöst, daß sie ohne Zerstörung der luftdicht-machenden Beschichtung bei Kochtemperaturen waschbar und/oder unter Heißdampfeinsatz sterilisierbar ist.

Der untere Zuschnitt besteht vorzugsweise aus einem Baumwoll- oder Leinengewebe und der obere Zuschnitt aus einer wasserabweisenden mit Kunststoff beschichteten Textilbahn aus Baumwolle und/oder Polyester. Dabei können die Textilbahnen aus einem Gewebe, einem Gewirke oder einem Vlies bestehen.

Der untere Zuschnitt sollte vorzugsweise aus einem Baumwoll- oder Leinengewebe bestehen, das ein Flächengewicht zwischen 80 und 250 g/m² besitzt. Es hat sich herausgestellt, daß ein solches Material ohne weiteres ohne zusätzliche Perforationen einen ausreichenden Luftaustritt aus dem Hohlraum gewährleistet. Dabei ist es möglich, einfache Bindungen zu verwenden, beispielsweise eine Leinwand- oder Köperbindung.

Dabei kann die Bindung des Gewebes so eingestellt werden, daß die Luftdurchlässigkeit bei einem im Hohlraum herrschenden Druck von 2 mbar zwischen 30.000 und 200.000 cm³ Luft pro dm² Bahnflache und Minute liegt.

Für den oberen Zuschnitt wird ebenfalls ein Gewebe, Gewirke oder Vlies verwendet, wobei hier der Luftdurchtritt natürlich nicht vorhanden ist, da der obere Zuschnitt beschichtet ist. Hier eignen sich insbesondere Polyester-, Polyurethan- oder hoch-hitzebeständige Elastomer-Verbindungen. Vorzugsweise liegt das Flächengewicht der textilen Bahn für den oberen Zuschnitt ohne Beschichtung zwischen 60 und 120 g/m² und mit Beschichtung zwischen 100 und 300 g/m².

Es sollte darauf geachtet werden, daß die Einfärbungen oder Griffeigenschaften, d. h. die optische und/oder haptische Unterscheidbarkeit der Zuschnitte deutlich ausfällt, so daß auch unter dem Streß des Operationsbetriebs immer leicht erkennbar ist, welche die untere und welche die obere Seite ist.

Die Lufteintrittsstutzen sollten ebenfalls den eingangs genannten Wasch- und Sterilisierungsbedingungen genügen. Hierbei hat sich eine Lösung anwendungstechnisch günstig erwiesen, bei der die Lufteintrittsstutzen als Manschetten gestaltet sind, in die ein Schlauchende eines Heißluft-Ventilators schiebbar ist. Die Manschettenenden besitzen einen in einem Hohlsaum der Manschette befindlichen Kordelzug, so daß sich ein ausreichend fester Anschluß des Schlauchs an die Lufteintrittsstutzen herstellen läßt.

Schließlich ist es noch möglich, die beiden Zuschnitte im Bereich des Hohlraums über Steppnähte miteinander zu verbinden, die den Hohlraum gliedern. Hierzu eignen sich übliche Garn-Nähte. Es ist aber auch nicht auszuschließen, über Verschweißungen oder Verklebungen Steppnähte herzustellen.

Die randseitige Verbindung der beiden Zuschnitte sollte vorzugsweise über eine dichte Garn-Naht erfolgen.

Ein Ausführungsbeispiel der Erfindung ist in der Zeichnung dargestellt und wird nachfolgend näher beschrieben. Es zeigen:
- Fig. 1: eine Draufsicht auf eine Wärmedecke im ausgebreiteten Zustand und
- Fig. 2: ein Detail im Bereich der Lufteintrittsstutzen.

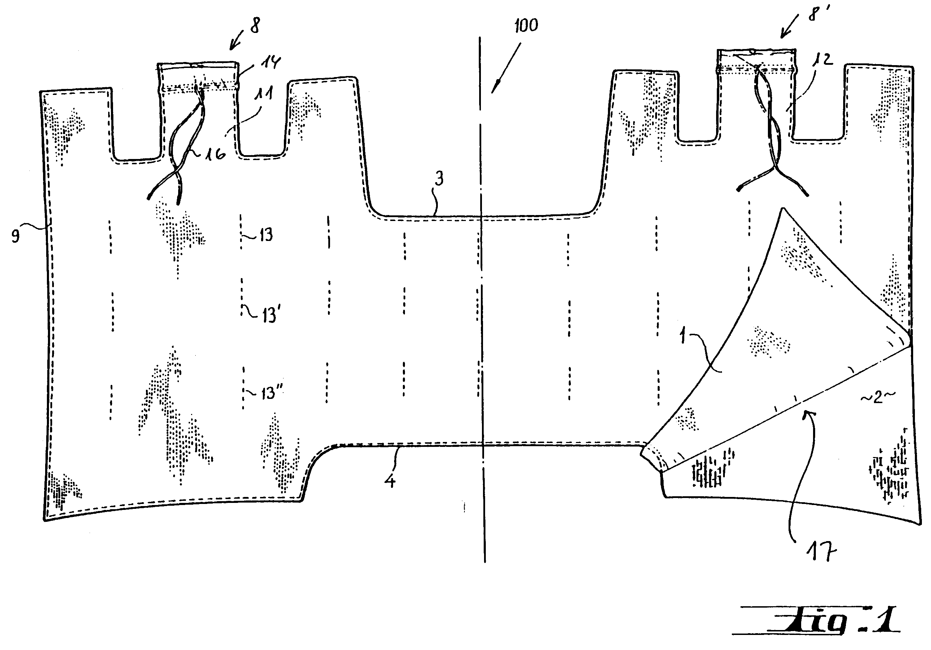

Die in Figur 1 dargestellte Wärmedecke 100 zur Verwendung bei ärztlichen Operationen hat eine axiale Ausdehnung, die ihrem Zwecke entspricht, nämlich die in ausgestreckter Armstellung liegenden Arme einschließlich Brustkorb eines Patienten zu überdecken. Der obere Ausschnitt 3 läßt Hals- und Kopfbereich frei, während der untere Ausschnitt 4 etwa in Höhe des Nabels endet, um den Bauchbereich freizulassen. Es sei aber darauf hingewiesen, daß auch andere Konfigurationen, wie sie an sich in der Operationstechnik bekannt sind, für die Wärmedecken gewählt werden können.

Die Wärmedecke besteht aus zwei randseitig über eine Naht 9 verbundene, im wesentlichen gleiche textile Zuschnitte 1 und 2, die zwischen sich einen Hohlraum 17 aufspannen, wenn beispielsweise Warmluft über ein Gebläse in den Hohlraum 17 eingeblasen wird, die unter einem Überdruck von 0,1 bis 2 mbar steht.

Die Zuschnitte 1 und 2 bestehen aus flexiblem Material, hier beispielsweise der untere, dem Patienten zugewandte Zuschnitt 2 aus einem Baumwoll- oder Leinengewebe, während der obere Zuschnitt 1 ein Polyester-getränktes oder beschichtetes Vliesmaterial oder ein Polyurethan-beschichtetes Polyterephthalsäureester ("Polyester") - Gewirke oder Gewebe ist. Wesentlich ist, daß das Material möglichst leicht und anschmiegsam ist. Es sollte den Patienten nicht belasten, aber trotzdem so beschaffen sein, daß die Wärmedecke 100 ohne Zerstörung der luftdicht-machenden Beschichtung bei Kochtemperaturen gewaschen werden kann und/oder bei Temperaturen bis zu 150° C unter Heißdampfeinsatz sterilisierbar ist. Letzteres geschieht üblicherweise in einem Autoklaven.

Wesentlich ist weiterhin, daß das Material des unteren Zuschnitts 2 eine definierte Luftdurchlässigkeit besitzt, um die Warmluft gleichmäßig verteilt nach unten auszulassen. Hierbei hat sich eine Luftdurchlässigkeit bewährt, die bei einem im Hohlraum 17 herrschenden Überdruck von 2 mbar zwischen 30.000 und 200.000 cm³ Luft pro dm² Bahnfläche und Minute hindurchläßt.

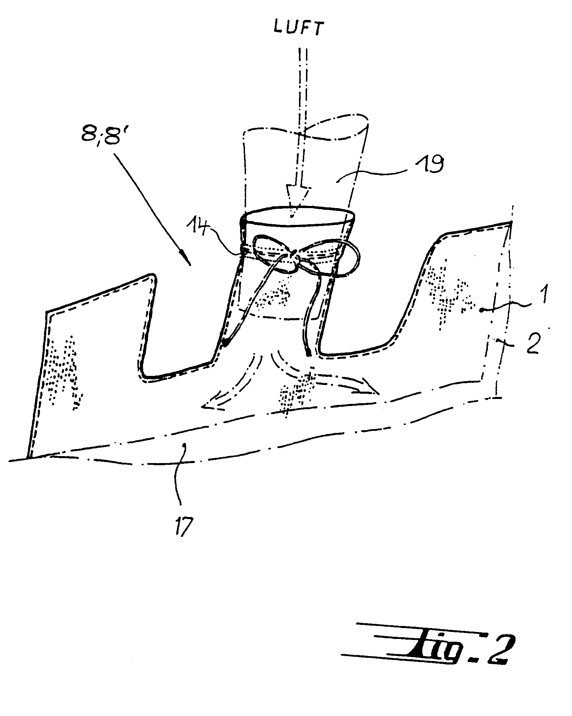

Um von beiden Patientenseiten her je nach Operationssituation die Wärmedecke 100 wahlweise mit Luft beschicken zu können, ist diese mit zwei Lufteintrittsstutzen 8, 8' versehen. Die Lufteintrittsstutzen 8, 8' sind, wie Figur 2 im Detail ausweist, als Manschetten 11, 12 gestaltet, die von einem Hohlsaum 14 umgeben sind. In den Hohlsaum 14 ist eine Kordel 16 eingeführt, die es ermöglicht, den von einem Ventilator (nicht dargestellt) kommenden Schlauch 19, der raumtemperierte oder warme Luft von etwa 46° C zuführt, ausreichend dicht zu umschließen.

Die beiden Zuschnitte 1, 2 sind verschieden eingefärbt, beispielsweise hellviolett und dunkelviolett, so daß leicht sicherzustellen ist, daß jeweils die richtige Seite dem Patientenkörper zugewandt aufgelegt wird.

Wie aus Fig. 1 weiterhin ersichtlich ist, ist die Wärmedecke 100 noch mit Steppnähten 13, 13', 13'' versehen, die den Hohlraum 17 gliedern, so daß sich die Luft gleichmäßig verteilt. Anstelle von Steppnähten 13, 13', 13'' können die Zuschnitte 1, 2 auch mit Schweißpunkten aufeinandergeheftet sein.

Ferner ist in Fig. 1 in der rechten Ecke ein Teil des oberen Zuschnitts 1 weggeschlagen, so daß der untere Zuschnitt 2 ersichtlich ist. Hier ist natürlich festzustellen, daß bei der fertiggestellten Wärmedecke 100 dieser Aufschlag über die umlaufende Naht 9 mit dem unteren Zuschnitt 2 verbunden ist.

### Aufstellung der Bezugszeichen:

- 1: oberer Zuschnitt
- 2: unterer Zuschnitt
- 3: oberer Ausschnitt
- 4: unterer Auschnitt
- 8: Lufteintrittsstutzen
- 8': Lufteintrittsstutzen
- 9: Naht
- 11: Manschette
- 12: Manschette
- 13: Steppnaht
- 13': Steppnaht
- 13'': Steppnaht
- 14: Hohlsaum
- 16: Kordel
- 17: Hohlraum
- 19: Schlauch
- 100: Wärmedecke

## Patentansprüche

1. Wärmedecke (100) zur Verwendung bei ärztlichen Operationen, die auf einen Teil eines liegenden Patienten aufzulegen und die mit temperierter Luft beschickbar ist, bestehend aus zwei randseitig zu einem Hohlraum (17) verbundenen Zuschnitten (1, 2) aus flexiblem Material,
- von denen der oben liegende Zuschnitt (1) aus einer luftdicht mit Kunststoff beschichteten textilen Bahn,
- und der dem Patienten zugewandte untere Zuschnitt (2) aus einem dichten, jedoch noch luftdurchlässigem textilen Gewebe besteht, wobei die im Hohlraum (17) unter leichtem Überdruck befindliche Luft aus der Unterseite der Wärmedecke (100), d. h. dem unteren Zuschnitt (2), flächenhaft verteilt austritt,
- und die mit wenigstens einem, vorzugsweise zwei im Abstand angeordneten, wechselseitig verschließbaren Lufteintrittsstutzen (8, 8') zum Anschluß an ein Luftgebläse versehen ist,
dadurch gekennzeichnet, daß der untere Zuschnitt (2) aus einem Baumwoll- oder Leinengewebe und der obere Zuschnitt (1) aus einer wasserabweisenden, kunststoffbeschichteten textilen Bahn aus Polyester und/oder Baumwolle besteht, so daß die Wärmedecke (100) ohne Beeinträchtigung der Funktion der luftdicht-machenden Beschichtung bei Kochtemperaturen waschbar und/oder unter Heißdampfeinsatz sterilisierbar ist.

2. Wärmedecke nach Anspruch 1, dadurch gekennzeichnet, daß die Textilbahnen des unteren Zuschnitts (2) und/oder des oberen Zuschnitts (1) aus einem Gewebe, Gewirke oder Vlies bestehen.

3. Wärmedecke nach Anspruch 1, dadurch gekennzeichnet, daß die Beschichtung des oberen Zuschnitts (1) aus einem Polyester, einem Polyurethan oder einem Elastomer besteht.

4. Wärmedecke nach einem der Ansprüche 1 bis 3, dadurch gekennzeichnet, daß das Flächengewicht der textilen Bahn für den oberen Zuschnitt (1) ohne Beschichtung zwischen 60 und 120 g/m² und mit Beschichtung zwischen 100 und 300 g/m² liegt.

5. Wärmedecke nach einem der Ansprüche 1 bis 4, dadurch gekennzeichnet, daß das Flächengewicht des Materials des unteren Zuschnitts (2) zwischen 80 und 250 g/m² liegt.

6. Wärmedecke nach einem der Ansprüche 1 bis 5, dadurch gekennzeichnet, daß der untere Zuschnitt (2) in Leinwand- oder Köperbindung gewebt ist.

7. Wärmedecke nach einem der Ansprüche 1 bis 6, dadurch gekennzeichnet, daß die Luftdurchlässigkeit bei einem im Hohlraum (17) herrschenden Druck von 2 mbar zwischen 30.000 und 200.000 cm³ Luft pro min und dm² Bahnfläche liegt.

8. Wärmedecke nach einem der Ansprüche 1 bis 7, dadurch gekennzeichnet, daß die Lufteintrittsstutzen (8, 8') als Manschetten (11, 12) gestaltet und mit einer Kordel (16) verschließbar sind.

9. Wärmedecke nach einem der Ansprüche 1 bis 8, dadurch gekennzeichnet, daß die beiden Zuschnitte (1, 2) im Bereich des Hohlraums (17) zu dessen Gliederung miteinander verbunden sind.

10. Wärmedecke nach einem der Ansprüche 1 bis 9, dadurch gekennzeichnet, daß der obere und der untere Zuschnitt (1, 2) optisch und/oder haptisch unterscheidbar sind.

## Claims

1. Heating cover (100) for use in medical operations, which is to be laid onto a part of a lying patient and chargeable with tempered air and which consists of two blanks (1, 2) of flexible material, which are connected at the rim into a hollow space (17),
- of which the blank (1) lying at the top consists of an airtight textile web coated with synthetic material,
- and the lower blank (2) facing the patient consists of a close, yet still air-permeable textile weave, wherein the air situated in the hollow space (17) under slight excess pressure issues out areally distributed from the underside of the heating cover (100), i.e. from the lower blank (2),
- and which is provided with at least one air entry connecting piece (8, 8'), preferably two arranged at a spacing and closable alternately, for connection to an air blower,
characterised thereby, that the lower blank (2) consists of a cotton or linen web and the upper blank (1) consists of a water-repellent textile web of polyester and/or cotton and coated with synthetic material so that the heating cover (100) is washable at boiling temperatures and/or sterilisable with the use of hot steam without impairing the function of the air-tight coating.

2. Heating cover according to claim 1, characterised thereby, that the textile webs of the lower blank (2) and/or of the upper blank (1) consist of a weave, knitted fabric or fleece.

3. Heating cover according to claim 1, characterised thereby, that the coating of the upper blank (1) consists of a polyester, a polyurethane or an elastomer.

4. Heating cover according to one of the claims 1 to 3, characterised thereby, that the weight per unit area of the textile web for the upper blank (1) without coating lies between 60 and 120 grams per square metre and with coating lies between 100 and 300 grams per square metre.

5. Heating cover according to one of the claims 1 to 4, characterised thereby, that the weight per unit area of the material of the lower blank (2) lies between 80 and 250 grams per square metre.

6. Heating cover according to one of the claims 1 to 5, characterised thereby, that the lower blank (2) is woven in basket weave or twill weave.

7. Heating cover according to one of the claims 1 to 6, characterised thereby, that the air permeability lies between 30,000 and 200,000 cubic centimetres of air per minute and square decimetre of web area at a pressure of 2 millibars prevailing in the hollow space (17).

8. Heating cover according to one of the claims 1 to 7, characterised thereby, that the air entry connecting pieces (8, 8') are structured as sleeves (11, 12) and closable by a cord (16).

9. Heating cover according to one of the claims 1 to 8, characterised thereby, that both the blanks (1, 2) are connected together in the region of the hollow space (17) for its formation.

10. Heating cover according to one of the claims 1 to 9, characterised thereby, that the upper blank (1) and the lower blank (2) are distinguishable optically and/or tactually.

## Revendications

1. Couverture chauffante (100) pour utilisation dans des opérations chirurgicales, que l'on place sur une partie d'un patient allongé et qui peut recevoir de l'air tempéré, cette couverture étant composée de deux éléments (1, 2) en matériaux flexibles, reliés le long de deux bords pour créer un volume creux (17) et dans laquelle :
• l'élément (1) situé au-dessus est fait d'un support textile recouvert d'une couche de matière plastique étanche à l'air.
• l'élément (2) situé en dessous et en regard du patient est fait d'un tissu textile serré, laissant toutefois passer l'air, de manière que l'air se trouvant dans le volume creux (17) et en légère surpression sorte à travers la face inférieure de la couverture chauffante (100), c'est-à-dire à travers l'élément inférieur (2), avec distribution sur toute la surface
• au moins une, de préférence deux tubulures d'entrée d'air (8, 8') espacées l'une de l'autre et obturables alternativement, sont prévues pour raccordement à un ventilateur,
caractérisée en ce que
l'élément inférieur (2) est fait d'un tissu de lin ou de coton tandis que l'élément supérieur (1) est fait d'un support textile en polyester et/ou en coton recouvert d'une couche hydrophobe en matière plastique, la couverture (100) pouvant, sans que soit portée atteinte au fonctionnement de la couche assurant l'étanchéité à l'air à des températures d'ébullition, être lavée et/ou stérilisée à la vapeur surchauffée.

2. Couverture selon la revendication 1,
caractérisée en ce que
le support textile de l'élément inférieur (2) et/ou de l'élément supérieur (1) est un tissu, un tricot ou une nappe.

3. Couverture selon la revendication 1,
caractérisée en ce que
la couche de revêtement de l'élément supérieur (1) est en polyester, en polyuréthanne ou en élastomère.

4. Couverture selon l'une des revendications 1 à 3,
caractérisée en ce que
la densité superficielle du support textile de l'élément supérieur (1) est de 60 à 120 g/m² sans le revêtement, de 100 à 300 g/m² avec le revêtement.

5. Couverture selon l'une des revendications 1 à 4,
caractérisée en ce que
la densité superficielle du matériau de l'élément inférieur (2) est comprise entre 80 et 250 g/m².

6. Couverture selon l'une des revendications 1 à 5,
caractérisée en ce que
l'élément inférieur (2) est un tissé, avec une armure de serge ou de toile.

7. Couverture selon l'une des revendications 1 à 6,
caractérisée en ce que
la perméabilité à l'air est de 30.000 à 200.000 cm³ par minute et par dm² de surface du support textile, pour une surpression de 2 mbars régnant dans le volume creux.

8. Couverture selon l'une des revendications 1 à 7,
caractérisée en ce que
les tubulures d'entrée d'air (8, 8') ont la forme de manchettes qu'un cordon (16) permet de fermer.

9. Couverture selon l'une des revendications 1 à 8,
caractérisée en ce que
les deux éléments (1, 2), pour assurer leur structuration, sont reliés entre eux dans la zone du volume creux (17).

10. Couverture selon l'une des revendications 1 à 9,
caractérisée en ce que
l'élément supérieur (1) et l'élément inférieur (2) peuvent être distingués par la vue et par le toucher.
